## Europäisches Patentamt
## European Patent Office
## Office européen des brevets

(11) Numéro de publication : **0 056 018**
**B1**

(12) **FASCICULE DE BREVET EUROPÉEN**

(45) Date de publication du fascicule du brevet :
09.10.85

(51) Int. Cl.⁴ : **G 01 N 33/546**, G 01 N 33/68, A 01 K 61/00

(21) Numéro de dépôt : 82400001.2

(22) Date de dépôt : 04.01.82

(54) **Procédé de reconnaissance des femelles en vitellogénèse chez les animaux ovipares, par un test d'immunoagglutination.**

(30) Priorité : 06.01.81 FR 8100103

(43) Date de publication de la demande :
14.07.82 Bulletin 82/28

(45) Mention de la délivrance du brevet :
09.10.85 Bulletin 85/41

(84) Etats contractants désignés :
AT BE CH DE GB IT LI LU NL SE

(56) Documents cités :
CHEMICAL ABSTRACTS, vol. 94, no. 19, 11 mai 1981, page 321, ref. 152971w, Columbus, Ohio, US LE BAIL et al.: "Rapid determination of the sex of puberal salmonid fish by a technique of immunoagglutination"
CHEMICAL ABSTRACTS, vol. 91, no. 9, 27 août 1979, page 300, résumé 71220m, Columbus, Ohio, US D.R. IDLER et al.: "Quantification of vitellogenin in Atlantic salmon (Salmo salar) plasma by radioimmunoassay"
CHEMICAL ABSTRACTS, vol. 83, no. 1, 7 juillet 1975, page 480, résumé 5474f Columbus, Ohio, US A. TANAKA et al.: "Immunochemical detection of vitellogenin in the ovary and fat body during a reproductive cycle of the cock-roach, Blattella germanica"
CHEMICAL ABSTRACTS, vol. 86, no. 7, 14 février 1977, page 218, résumé 39689g Columbus, Ohio, US M.R. REDSHAW et al.: "Physiology of egg yolk production by the fowl: the measurement of circulating levels of vitellogenin employing a specific radioimmunoassay"
I.M. Roitt "Essential Immunology" 4th edition 1980 pages 152-156

(73) Titulaire : **INSTITUT NATIONAL DE LA RECHERCHE AGRONOMIQUE (INRA)**
**149, rue de Grenelle**
**F-75341 Paris Cedex 07 (FR)**

(72) Inventeur : **Le Bail, Pierre Yves**
**13 rue des 3 Epis**
**F-38100 Grenoble (FR)**
Inventeur : **Breton, Bernard**
**24 rue George Sand**
**F-35550 Cesson-Thorigne Sur Vilaine (FR)**
Inventeur : **Maisse, Gérard**
**17 rue de la Jaunaie**
**F-35550 Cesson-Thorigne Sur Vilaine (FR)**

(74) Mandataire : **Gillard, Marie-Louise et al**
**Cabinet Beau de Loménie 55, Rue d'Amsterdam**
**F-75008 Paris (FR)**

Jouve, 18, rue St-Denis, 75001 Paris, France

## Description

L'invention a pour objet un test simple, rapide et spécifique permettant de reconnaître le sexe des animaux ovipares, sans léser ceux-ci.

La reconnaissance du sexe, notamment chez les poissons, est utile aux écologistes pour déterminer le potentiel producteur des populations. Elle peut permettre également d'optimiser la gestion des stocks de géniteurs destinés à la production piscicole.

En dehors de la période de reproduction, la recherche des critères de sexage à partir de caractères morphologiques est une opération longue et hasardeuse. Par contre, il est possible d'envisager une technique de sexage fondée sur la reconnaissance biochimique d'une protéine spécifique de l'un des sexes.

Dès 1932, SASAKI proposait de reconnaître le sexe des poulets en utilisant comme critère de sexage la présence d'une protéine plasmatique précurseur des réserves vitellines de l'œuf. Plus récemment, une protéine équivalente (la vitellogénine) a été mise en évidence chez les saumons et chez la truite grâce à des techniques d'électrophorèse et d'immunodiffusion.

Le procédé décrit par IDLER et al. (« J. Fish-RES. Board. Can » 1979, 36 (5), pages 574-8) consiste en la détection de la vitellogénine plasmatique à partir d'un dosage hétérologue, dans lequel l'anticorps et la protéine marquée sont produits grâce à une phosvitine de l'œuf dérivée mais totalement distincte de la vitellogénine plasmatique. Dans ce cas, les propriétés communes de la phosvitine et de la vitellogénine sont mises en œuvre ; mais un tel dosage et les valeurs qui en découlent sont très critiquables et ne constituent pas à proprement parlé un dosage spécifique et absolu de la vitellogénine. D'autre part l'anticorps mis en œuvre dans ce procédé n'est pas un anticorps anti-vitellogénine mais anti-vitellus de saumon.

Bien que suffisamment sensible pour autoriser la détection de la vitellogénine au cours du cycle annuel, la difficulté de leur mise en œuvre sur le terrain ne permet pas de considérer ces techniques comme susceptibles de fournir des réponses immédiates aux utilisateurs potentiels.

Le procédé de sexage selon l'invention consiste à déceler la vitellogénine présente spécifiquement dans le sang des femelles à un certain moment du cycle de reproduction, c'est-à-dire en cours de vitellogénèse, par un test d'immunoagglutination effectué sur un échantillon de sérum ou de sang de l'animal ; cet échantillon est mis en présence d'un support sensibilisé par des anticorps antivitellogénine, et la formation d'un précipité (test positif) indique la présence de vitellogénine.

Le test d'immunoagglutination est basé sur la réaction entre l'antigène (vitellogénine dans le milieu à tester) et son anticorps spécifique fixé sur un support ayant une surface spécifique relativement importante tel que des hématies tannées de mouton ou des particules de latex.

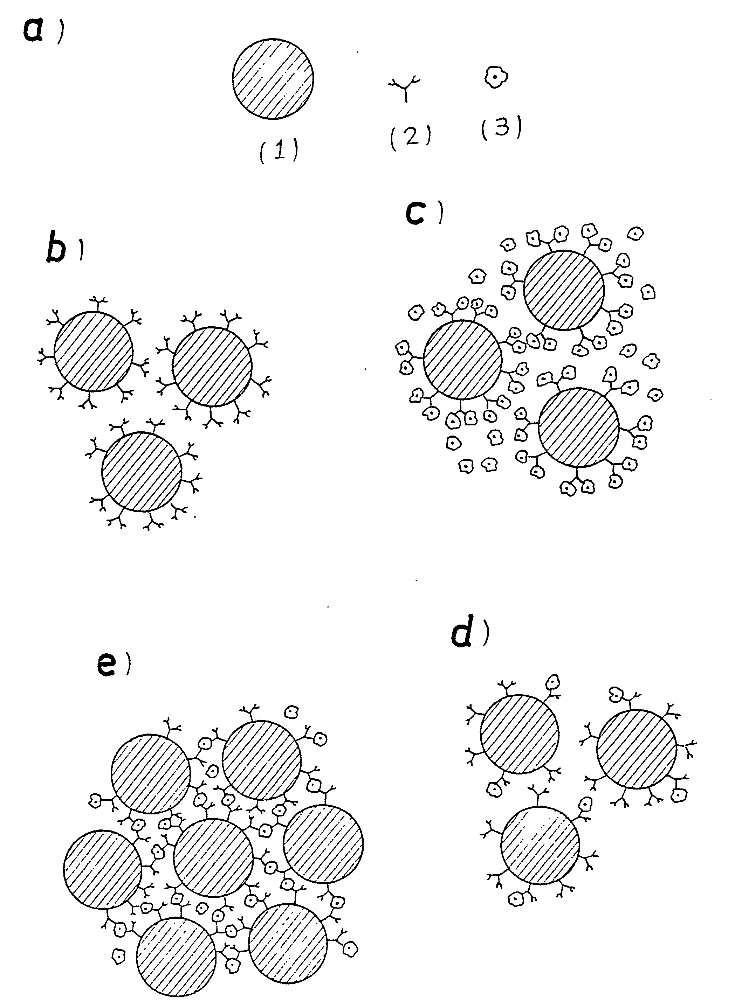

Le support a pour but de rendre visible la réaction antigène-anticorps. Bien que celle-ci ait toujours lieu quel que soit le rapport des concentrations antigène-anticorps, elle n'est visible que dans une plage réduite des rapports de concentration dite « zone d'équivalence ». En effet, la visualisation de la réaction correspond à une précipitation du complexe antigène-anticorps due à la formation d'une densité suffisante de ponts entre les anticorps par les antigènes. La figure en annexe illustre ce phénomène.

Sur cette figure on a représenté :

en a) les différents produits en présence : 1) le support (billes de latex), 2) l'anticorps, 3) l'antigène ;

en b) le support sensibilisé, c'est-à-dire revêtu de l'anticorps ;

en c) le cas où l'antigène est en excès : il y a saturation par l'antigène des sites de liaison (anticorps) sur le support ;

en d) le cas où l'anticorps est en excès : il n'y a que très peu de sites de liaison anticorps-antigène sur le support.

Dans les deux cas c) et d) où l'un des réactifs est en excès, la densité des ponts formés est trop faible pour former un réseau précipitant, et la réaction apparaît négative à l'observateur.

Par contre en e), qui représente ce qui se passe dans la « zone d'équivalence », le nombre de ponts formés entre les anticorps par les antigènes est suffisant pour provoquer une précipitation (agglutination) visible : la réaction apparaît positive.

Par conséquent, pour une concentration donnée d'anticorps sur le support une réaction apparemment négative peut résulter d'une absence d'antigène, mais aussi d'une concentration d'antigène en excès dans le milieu testé. Dans ce cas, pour trancher entre ces deux possibilités (absence ou excès d'antigène) on ajoute aux réactifs déjà en présence du sérum connu, donnant une réaction positive (c'est-à-dire contenant l'antigène) : si une précipitation se produit, il y avait absence d'antigène dans le milieu testé. Dans le cas contraire, l'antigène était en excès par rapport à la concentration d'anticorps choisie.

Pour mettre en œuvre le test d'agglutination il faut disposer de l'anticorps spécifique de la vitellogénine avec lequel on sensibilisera un support adéquat. Les antisérums contenant les $\gamma$-globulines antivitellogénine (anti-VgP) ont été préparés par injection à des lapins de vitellogénine pure (VgP) isolée à partir du sang de poissons (en particulier de salmonidés) œstrogénisés.

Obtention de vitellogénine pure :

Chez les salmonidés, l'œstrogénisation du mâle et de la femelle, quelle que soit l'époque du cycle reproducteur, induit une synthèse hépatique de vitellogénine et une augmentation de la concentration plasmatique de cette protéine (BAILEY, 1957, TAKASHIMA and al., 1972). Cette méthode a été appliquée à des truites arc-en-ciel femelles qui ont été traitées par de l'œstradiol-17β en injection intra-péritonéale pendant quatre semaines, à raison d'une injection de 0,5 mg/kg tous les deux jours. A la fin du traitement, les animaux ont été sacrifiés et le sang est recueilli au niveau de l'artère caudale. La purification de la vitellogénine est inspirée de la technique de HARA et HIRAI (1978) : après coagulation le sérum est séparé par centrifugation et mis à dialyser trois jours contre cent volumes d'eau distillée renouvelée chaque jour. Le précipité formé est séparé par centrifugation (3 000 g, 4 °C, une heure). Le culot est redissous dans un tampon tris 0,05 M NaCl M, pH 9,2. L'insoluble est éliminé par centrifugation. Le surnageant est déposé sur une colonne de sépharose® 6B (1m × 1,6 cm).

La reconnaissance de la vitellogénine dans les fractions issues de la chromatographie a été faite par électrophorèse sur gel de polyacrylamide à 5 % (14 cm de long) selon la technique de ORNSTEIN et DAVIS (1964) modifiée en réajustant le pH du tampon de migration à 9,2 par NaOH N. Des sérums de femelles en vitellogénèse et de mâles sont utilisés comme référence. Les fractions les plus riches sont réunies et dialysées comme de l'eau distillée. Le précipité est repris dans le tampon tris 0,05M NaCl M pH 9,2, puis rechromatographié sur sépharose® 6B et dialysé dans les mêmes conditions que précédemment.

La concentration de la solution finale de vitellogénine (VgP) a été déterminée par la méthode de LOWRY et al. (1951).

### Préparation des γ-globulines antivitellogénine

On injecte à des lapins, par voie sous-cutanée, 0,5 mg de VgP en présence d'adjuvant de Freund complet, et répète cette injection tous les quinze jours pendant 3 mois. Après cette période les animaux sont saignés, et on isole les γ-globulines anti-VgP à partir du sérum par précipitation au sulfate d'ammonium à 33 % de saturation ; le précipité est centrifugé, dialysé contre de l'eau distillée et lyophilisé.

### Préparation du support sensibilisé :

On a utilisé des billes de latex (Difco 0,81), mais on peut utiliser tout autre support d'adsorption classique. Les γ-globulines anti-VgP lyophilisées sont redissoutes dans un tampon véronal (0,05 M) CaCl₂ (5 mM). La fixation de l'anticorps sur les billes de latex s'effectue selon la technique de McCARTHY (1975) dans lequel le tampon glycine-NaCl a été remplacé par un tampon véronal pH 8,2. Les essais ont été faits en sensibilisant les billes par des solutions contenant les γ-globulines anti-VgP à des concentrations 4 et 9 fois supérieures à la concentration des γ-globulines dans le sérum.

### Test d'agglutination :

Le test d'agglutination s'effectue en mettant en présence, sur une lame de verre dégraissée, une goutte du milieu à tester, une goutte de tampon véronal pH 8,2 et une goutte de la suspension de billes de latex sensibilisée. Le mélange est homogénéisé et agité lentement. La réaction positive, caractérisée par l'apparition d'aggrégats de billes, se développe en 2 à 3 minutes. Quand la réaction est négative, on ajoute du sérum positif témoin et attend encore 1 à 2 minutes, pour déterminer si on est en présence d'un excès (réponse négative) ou d'une absence de vitellogénine (réponse positive).

On n'a observé aucune différence entre les réactions du sérum et du plasma d'un même animal. On peut également utiliser le sang entier pour effectuer le test. Cependant, le sang provoquant des coagulations qui peuvent être confondues avec l'agglutination spécifique, on élimine cette difficulté en ajoutant de l'héparine (300 UI/ml) au tampon de réaction. Pour une même réponse, il faut utiliser une quantité de sang de 1 fois et demie à 2 fois plus grande que celle des sérums correspondants.

### Validation du test :

La validation des réponses au test d'agglutination a été recherchée d'une part par immunodiffusion sur gel d'agarose à 0,8 % en tampon véronal 0,05 M, pH 8,3, et d'autre part en effectuant le test sur le plasma d'animaux préalablement sexés par observation des gonades. Les essais ont portés sur deux espèces :

Les truites fario (Salmo trutta) : des mâles et femelles ont été abattus mensuellement au cours de leur premier cycle reproducteur. Les femelles ont été caractérisées soit à partir des rapports gonadosomatique (RGS) soit à partir du stade précis et du diamètre des ovocytes au cours des phases finales du cycle, selon les critères définis par JALABERT (1978) : fin de vitellogénèse (FV), maturante, ovulée. Du plasma de chaque animal a été obtenu après prélèvement de sang avant abattage. Ce plasma est conservé congelé jusqu'au test.

Les saumons atlantique : proviennent de captures par des pêcheurs sportifs pendant toute la période de pêche légale. Le sang total a été congelé sur le terrain, le sérum n'étant séparé par centrifugation qu'après son arrivée au laboratoire. Les animaux ont été caractérisés par leur rapport gonado-somatique, et par le diamètre moyen des ovocytes calculé à partir de 48 ovocytes pris sur six lamelles ovigères différentes.

Sensibilité du test :

Sur le tableau I ci-après, on a porté, pour des concentrations en vitellogénine variant de 120 à 0,05 mg/ml, les réponses obtenues d'une part avec le test d'immunoagglutination selon l'invention en utilisant deux concentrations de γ-globulines pour sensibiliser les billets de latex, et d'autre part avec le test d'immunodiffusion.

Le tableau montre qu'aux deux concentrations d'anti-VgP de sensibilisation (9 fois et 4 fois plus grande que celle de l'antisérum entier) correspondent deux gammes différentes de concentration en vitellogénine conduisant à des réponses positives. La sensibilité de réponse pour des milieux à faible concentration en vitellogénine est augmentée en diminuant la quantité d'anticorps sensibilisants.

Des deux côtés de la plage de réponse positive, une réaction négative peut être due soit à un excès, soit à un manque de vitellogénine ; d'où la nécessité dans le cas d'une réaction négative, de l'addition au milieu réactionnel d'un sérum connu positif, pour trancher entre les deux hypothèses.

Le test d'agglutination selon l'invention a été appliqué à la détection de la vitellogénine au cours du cycle reproducteur des deux espèces de poisson indiquées ci-dessus.

Truite fario : Les résultats obtenus sont portés dans le tableau II ci-après.

A toute époque du cycle aucune réaction positive n'a été mise en évidence à partir des sérums mâles.

Par contre, chez la femelle, l'apparition de vitellogénine peut être détectée chez quelques animaux à partir du 12 avril et devient générale le 26 mai, alors que la phase de croissance rapide de la gonade et des ovocytes n'a pas débuté. Durant une période d'un ou deux mois post-ovulation, aucune réponse positive n'a été obtenue. Sur un échantillon plus important le test d'immunodiffusion a permis de confirmer que toutes les femelles possèdent de la vitellogénine circulante aux environs de la mi-mai.

Saumon atlantique : Les captures se sont échelonnées de fin mars à fin juin, époque à laquelle aucun caractère sexuel secondaire ne permet de différencier les sexes.

La vitellogénine est détectée chez toutes les femelles par immunoagglutination, alors que les RGS sont faibles et que les diamètres des ovocytes ne varient pas sensiblement durant la saison de pêche. Aucun des mâles n'a répondu positivement.

Ainsi, l'étude réalisée sur un cycle complet chez la truite fario montre que la sensibilité du test d'immunoagglutination permet une détection qualitative de la vitellogénine dès le mois d'avril et constitue un procédé de sexage suffisamment sûr dès le mois de mai. L'utilisation du procédé selon l'invention permet donc la constitution des stocks de géniteurs femelles en pisciculture plusieurs mois avant l'apparition des caractères morphologiques utilisés généralement pour la reconnaissance des sexes.

Pour le saumon atlantique, des études antérieures signalent la présence de vitellogénine chez les femelles à différents stades à la remontée, et sur frayères. L'étude réalisée avec le test d'agglutination donne les mêmes résultats : toutes les femelles pêchées entre début mars et mi-juin répondent positivement ; la vitellogénèse serait donc engagée au moment de l'entrée en eau douce. La mise en œuvre du procédé selon l'invention au niveau des pièges de capture est donc tout indiquée pour déterminer le sexe ratio et le potentiel reproducteur d'une population de saumons.

Les concentrations d'anticorps sensibilisants ont permis d'obtenir des limites de détection, compatibles avec les taux de vitellogénine circulante chez la plupart des animaux entrant en vitellogénèse. Une sensibilité plus importante du test pourrait être obtenue en diminuant la quantité d'anticorps fixés sur les billes. Ces conditions devraient permettre d'étendre les plages de réponses positives vers les faibles concentrations en vitellogénine, et d'accroître les pourcentages de sexage en début de cycle. Il faut cependant préciser que plus le test est sensible plus les temps de réponse augmentent et moins nette est la lecture. Ainsi la définition des rapports optimaux, anticorps sensibilisants/support doit être déterminé dans chaque cas particulier.

(Voir tableaux pages suivantes)

# Tableau I

Gamme de réponse des tests d'immunoagglutination et d'immunodiffusion contre de la vitellogénine.

| Vitellogénine en mg/ml / Tests | 120 | 60 | 30 | 15 | 8 | 4 | 2 | 1 | 0,5 | 0,25 | 0,1 | 0,05 |
|---|---|---|---|---|---|---|---|---|---|---|---|---|
| **Immunoagglutination** | | | | | | | | | | | | |
| X 9 | - | - | (+) | + | + | + | + | (+) | - | - | - | - |
| X 4 | - | - | - | (+) | + | + | + | + | + | (+) | - | - |
| **Immunodiffusion** | | | | | | | | | | | | |
| sérum entier | + | + | + | + | + | + | + | + | (+) | | | |

+ réaction positive
− réaction négative
(+) limite de détection
X 9, X 4 : solution de billes de latex sensibilisées avec une concentration de γ-globuline 9 et 4 fois plus importante que celle de l'antisérum entier.

0 056 018

## Tableau II

### Présence de la vitellogénine au cours du cycle annuel de la truite fario femelle

| Nombre d'animaux | Date | RGS ($\bar{X} \pm \sigma$) | Ø W ($\bar{X} \pm \sigma$) | Stade | Concentration de l'anticorps (AVgP) X9 | | X4 | |
|---|---|---|---|---|---|---|---|---|
| | | | | | sérum dilué au 1/5 | sérum entier | sérum entier | addition de sérum positif de femelle |
| 5 | 02.11.1976 | 14,54 ± 1,34 | 4,27 ± 0,12 | F.V. | + (1) | + (4) | | |
| 5 | 16.11.1976 | 15,04 ± 2,02 | 4,40 ± 0,14 | F.V. | | + (5) | | |
| 6 | 30.11.1976 | 17,16 ± 2,26 | 4,90 ± 0,15 | F.V. | | + (6) | | |
| 5 | 13.12.1976 | 16,36 ± 3,19 | 4,43 ± 0,11 | maturante | | + (5) | | |
| 5 | 13.01.1977 | 1,61 ± 0,23 | < 1 | ovulée | | + (3) | - (2) | + (2) |
| 5 | 10.02.1977 | 0,87 ± 0,31 | < 1 | post-ovulée | | + (1) | +(1)-(3) | + (3) |
| 5 | 18.03.1977 | 0,85 ± 0,15 | < 1 | | | | - (5) | + (5) |
| 5 | 12.04.1977 | 0,85 ± 0,15 | < 1 | | | + (2) | - (3) | + (3) |
| 5 | 12.05.1977 | 0,56 ± 0,40 | < 1 | | | + (2) | - (3) | + (3) |
| 5 | 26.05.1977 | 1,02 ± 0,28 | < 1 | | | + (5) | | |
| 5 | 17.06.1977 | 1,17 ± 0,26 | 1,25 ± 0,18 | | | + (5) | | |
| 5 | 20.07.1977 | 2,56 ± 0,45 | 2,06 ± 0,33 | | | + (5) | | |
| 5 | 18.08.1977 | 3,81 ± 1,29 | 2,64 ± 0,42 | | + (2) | + (3) | | |
| 5 | 14.09.1977 | 10,46 ± 2,45 | 3,89 ± 0,40 | | + (3) | + (2) | | |
| 4 | 13.10.1977 | 14,21 ± 2,70 | 4,43 ± 0,15 | F.V. | + (3) | | - (1) | + (1) |
| 4 | 25.10.1977 | 17,39 ± 3,40 | 4,45 ± 0,26 | F.V. | + (4) | | | |

Les chiffres entre parenthèses correspondent au nombre de réponses
RGS : rapport gonado-somatique
Ø W : diamètre des ovocytes
F.V. : fin de la vitellogenèse

## Revendications

1. Procédé de reconnaissance des femelles en vitellogénèse chez les animaux ovipares consistant à déceler la vitellogénine présente spécifiquement dans le sang de celles-ci, caractérisé en ce qu'on effectue un test d'immunoagglutination sur un échantillon de sang ou de sérum de l'animal, que l'on met en présence d'un support sensibilisé par des gamma-globulines anti-vitellogénine, la formation d'un précipité dans l'échantillon testé indiquant la présence de vitellogénine.

2. Procédé selon la revendication 1, caractérisé en ce que, lorsque le test est négatif, l'addition ultérieure à l'échantillon testé de sérum connu contenant de la vitellogénine permet de trancher entre d'une part une absence/présence en faible concentration de vitellogénine (formation d'un précipité) et d'autre part un excès de vitellogénine (absence de précipité) dans l'échantillon initial.

3. Procédé selon l'une des revendications 1 et 2, caractérisé en ce que, lorsqu'on effectue le test sur un échantillon de sang, on ajoute de l'héparine au tampon de réaction.

4. Procédé selon l'une des revendications 1 à 3, caractérisé en ce que le support est sensibilisé, par un procédé connu en soi, à l'aide de solutions de gamma-globulines anti-vitellogénine de concentrations variables, la sensibilité du test étant d'autant plus grande pour les milieux pauvres en vitellogénine que cette concentration est plus faible.

5. Procédé selon la revendication 4, caractérisé en ce que la concentration des anticorps sensibilisants est de 4 à 9 fois plus grande que celle des anticorps dans l'antisérum entier.

6. Procédé selon l'une des revendications 1 à 5, caractérisé en ce qu'on détecte les femelles en vitellogénèse chez les poissons et en particulier chez les salmonidés.

7. Procédé selon l'une des revendications 1 à 6, caractérisé en ce que les gamma-globulines utilisées pour la sensibilisation du support sont obtenues de façon classique à partir d'un antisérum de lapins ayant reçu des injections de vitellogénine.

8. Procédé selon l'une des revendications 1 à 7, caractérisé en ce que la vitellogénine injectée aux lapins est extraite du sang de salmonidés œstrogénisés, et purifiée par chromatographie.

## Claims

1. Process for recognising females in the course of vitellogenesis in oviparous animals, consisting in detecting the vitellogenin specifically present in the blood of females, characterized in that an immunoagglutination test is carried out on a sample of blood or of serum of the animal, which is brought into presence with a support sensitised by anti-vitellogenin gamma-globulins, the formation of a precipitate in the sample tested indicating the presence of vitellogenin.

2. The process according to claim 1, characterized in that, when the test is negative, known serum containing vitellogenin is subsequently added to be tested sample to determine between, on the one hand an absence/presence in low concentration of vitellogenin (formation of a precipitate), and on the other hand, an excess of vitellogenin (absence of precipitate) in the initial sample.

3. The process according to any one of claims 1 and 2, characterized in that, when the test is carried out on a blood sample, heparin is added to the reaction buffer.

4. The process according to any one of claims 1 to 3, characterized in that the support is sensitised, by a process known per se, with the aid of solutions of anti-vitellogenin gamma-globulins of variable concentrations, the sensitivity of the test being greater for the media poor in vitellogenin as this concentration is weaker.

5. The process according to claim 4, characterized in that the concentration of the sensitising antibodies is 4 to 9 times greater than that of the antibodies in the whole antiserum.

6. The process according to any one of claims 1 to 5, characterized in that the females in the course of vitellogenesis in fish and in particular in the Salmonidae family are detected.

7. The process according to any one of claims 1 to 6, characterized in that the gamma-globulins used for sensitisation of the support are obtained in conventional manner from an antiserum from rabbits having received injections of vitellogenin.

8. The process according to any one of claims 1 to 7, characterized in that the vitellogenin injected in rabbits is extracted from the blood of oestrogenised Salmonidae, and purified by chromatography.

## Patentansprüche

1. Verfahren zum Erkennen von Weibchen bei der Dotterbildung bei eierlegenden Tieren durch Nachweis des spezifisch im Blut derselben vorhandenen Vitellogenins, dadurch gekennzeichnet, daß man an einer Blut- oder Serumprobe des Tieres einen Immunagglutinationstest vornimmt und in der getesteten Probe in Gegenwart eines mit Antivitellogenin-Gammaglobulinen sensibilisierten Trägers die Bildung eines Niederschlags bewirkt, der das Vorhandensein von Vitellogenin anzeigt.

2. Verfahren nach Anspruch 1, dadurch gekennzeichnet, daß bei einem negativen Test die anschließende Zugabe von bekannten, Vitellogenin enthaltendem Serum zur Probe ermöglicht, zwischen

einerseits dem Fehlen/Vorhandensein in geringer Konzentration von Vitellogenin (Bildung eines Niederschlags) und anderseits einem Überschuß an Vitellogenin (Fehlen eines Niederschlags) in der Ausgangsprobe zu unterscheiden.

3. Verfahren nach einem der Ansprüche 1 und 2, dadurch gekennzeichnet, daß man bei Durchführung des Tests an einer Blutprobe zum Reaktionspuffer Heparin zugibt.

4. Verfahren nach einem der Ansprüche 1 bis 3, dadurch gekennzeichnet, daß der Träger durch ein an sich bekanntes Verfahren mittels Lösungen von Antivitellogenin-Gammaglobulinen variabler Konzentrationen sensibilisiert wird, wobei die Sensibilität des Tests bei den vitellogeninarmen Medien umso größer ist, je geringer diese Konzentration ist.

5. Verfahren nach Anspruch 4, dadurch gekennzeichnet, daß die Konzentration der sensibilisierenden Antikörper 4-bis 9mal größer als jene der Antikörper im Antivollserum ist.

6. Verfahren nach einem der Ansprüche 1 bis 5, dadurch gekennzeichnet, daß man bei Fischen, insbesondere bei Lachsfischen, die Weibchen bei der Dotterbildung ausfindig macht.

7. Verfahren nach einem der Ansprüche 1 bis 6, dadurch gekennzeichnet, daß die zur Sensibilisierung des Trägers verwendeten Gammaglobuline auf klassische Weise aus dem Antiserum von Kaninchen, denen Vitellogenin injiziert wurde, gewonnen werden.

8. Verfahren nach einem der Ansprüche 1 bis 7, dadurch gekennzeichnet, daß das den Kaninchen injizierte Vitellogenin aus dem Blut von östrogenisierten Lachsfischen extrahiert und mittels Chromatografie gereinigt wird.